(19) Europäisches Patentamt
European Patent Office
Office européen des brevets

(11) **EP 4 370 185 B1**

(12) **EUROPEAN PATENT SPECIFICATION**

(45) Date of publication and mention
of the grant of the patent:
**21.08.2024 Bulletin 2024/34**

(21) Application number: **22743456.0**

(22) Date of filing: **29.06.2022**

(51) International Patent Classification (IPC):
**A61M 16/12** (2006.01)   **A61M 16/20** (2006.01)
**A61M 16/10** (2006.01)

(52) Cooperative Patent Classification (CPC):
**A61M 16/12; A61M 16/101; A61M 16/201;**
A61M 16/204; A61M 2016/102; A61M 2202/0208

(86) International application number:
**PCT/EP2022/067836**

(87) International publication number:
**WO 2023/285142 (19.01.2023 Gazette 2023/03)**

(54) **MULTI-MODE MEDICAL GAS DELIVERY WITH NON-INVASIVE VENTILATION**

MULTIMODALE MEDIZINISCHE GASABGABE MIT NICHTINVASIVER BEATMUNG

DISTRIBUTION DE GAZ MÉDICAL MULTIMODE AVEC VENTILATION NON INVASIVE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.07.2021 US 202163220595 P**

(43) Date of publication of application:
**22.05.2024 Bulletin 2024/21**

(73) Proprietor: **Koninklijke Philips N.V.
5656 AG Eindhoven (NL)**

(72) Inventors:
• **WHITCHER, Douglas, Adam
5656 AG Eindhoven (NL)**
• **SERVANSKY, Daniel, Paul
5656AG Eindhoven (NL)**

(74) Representative: **Philips Intellectual Property &
Standards
High Tech Campus 52
5656 AG Eindhoven (NL)**

(56) References cited:
EP-A2- 2 068 992      US-A1- 2009 183 737
US-A1- 2011 214 676      US-A1- 2019 344 033

**Description**

**CROSS-REFERENCE TO RELATED APPLICATIONS**

This patent application claims the priority benefit under 35 U.S. C. § 119(e) of U.S. Provisional Application No. 63/220,595, filed on July 12, 2021.

**FIELD OF THE PRESENT SYSTEM:**

The present system relates to a gas delivery system, such as a ventilation system, providing non-invasive gas delivery/ventilation and, more particularly, to a multi-mode gas delivery device, such as an oxygen therapy device, including continuous or pulse flow modes of medical gas with enhanced efficiency and portability, and a method of operation thereof.

**BACKGROUND OF THE PRESENT SYSTEM:**

Non-invasive open ventilation devices - (NOVDs), such as the TIDAL ASSIST VENTILATOR™ by Inogen™, are handheld devices which may deliver continuous or pulsed flow oxygen therapy, or oxygen enriched non-invasive ventilation (NIV) through an air entrainment delivery system, such as a cannula or mask. Air entrainment is a phenomenon that occurs when air/gas under pressure is released from a device in such a way that a low pressure is generated in the immediate area of a gas discharge near a patient interface. Gas from the surrounding environment is then pulled into the discharged air stream, increasing the volumetric flow rate that is delivered to the patient interface. These NOVDs require connection to a supplemental oxygen source such as an oxygen cylinder or stationary oxygen concentrator which are heavy and limit the portability and run times of these NOVDs. Additionally, the oxygen cylinder has a limited capacity and stationary oxygen concentrator requires mains power. This requirements of NOVDs may adversely impact individuals with mobile lifestyles that rely upon these systems for support.

Supplemental oxygen sources when used with conventional systems must typically deliver up to 5 liters per minute (LPM) of 25+ psi oxygen. This delivery requirement necessitates either the use of large oxygen cylinders which may weigh upwards of 15 pounds or the use of a stationary mains-powered oxygen concentrator, each of which qualities negate portability. Although battery-powered oxygen concentrators may meet some oxygen delivery requirements, these battery-powered units are heavy and suffer from high energy consumption which result in a short run time. These factors negate portability and may inconvenience users who depend upon these systems for support.

Run time vs mobility

Although portable ventilators may use oxygen (e.g., from an oxygen cylinder) as the gas source to drive an air entrainment interface when used in an ambulatory or stand-alone modes, this oxygen use may deplete available oxygen from the oxygen cylinder in a short time. More particularly, these devices consume oxygen during an entire inspiratory delivery pulse as well as during the expiratory phase which drastically reduces the amount of time that the oxygen cylinder will last. Accordingly, to remain mobile, a user may have to lug around several heavy oxygen cylinders to attain a desired mobile run time using these portable ventilators.

Although, using an oxygen cylinder, such as the standard M-9 cylinder, may provide for some level of portability, usage time is limited as the M-9 cylinder may provide less than 50 minutes of use time (e.g., run time). Conversely, a stationary oxygen concentrator would allow for unlimited use but without any portability beyond the length of a standard patient interface line set (e.g., such as a cannula line set).

Oxygen Therapy & Efficiency Thereof

NOVD devices may utilize purified oxygen as the only gas source to drive the air entrainment through the cannula. This use of oxygen is highly inefficient and results in inefficient Oxygen Capture Time (OCT), lung fill (LF), and Therapeutic Oxygen therapy as is discussed below.

Oxygen Capture Time

The ISO 80601-2-67 standard states that the Oxygen Capture Time (OCT) is three fifths of the Inhale Period (IP) (e.g., an inspiratory period). Likewise, the IP is the first 1/3 of the Breathing Period (BP). For example, if a patient has a respiratory rate of 20 Breaths per Minute (BrPM) then the BP is 3000 ms and the IP is 1000 ms. Of the IP, only 600 ms is the OCT and thus any oxygen delivered to the patient after the OCT should not be considered part of the volume of therapeutic oxygen delivered to the patient. Since conventional NOVD devices utilize purified oxygen as the only gas source to drive the air entrainment through the cannula, any oxygen that is delivered to the patient beyond the OCT is not therapeutic oxygen per the ISO standard.

Lung Fill

When inhaling, a patient draws air into the lungs to fill the bronchial tubes, bronchioles, alveoli and ultimately deliver oxygen to the gas exchange capillaries in the interstitium. Any oxygen which does not make it to capillaries is unused and exhaled. Since conventional NOVD devices utilize purified oxygen as the only gas source to drive the air entrainment through the cannula, any oxygen that is delivered and does not make it to the capillaries (i.e., left in the bronchioles, bronchial tubes, trachea, oral cavity, or nasal cavity) is exhaled and becomes wasted potential therapy gas, thus leading to a highly inefficient delivery method. This highly inefficient delivery method of prior systems wastes oxygen and depletes available oxygen supplies thus necessitating large and heavy oxygen cylinders or power wasting oxygen concentrators to generate therapeutic oxygen.

Oxygen Concentrators

Therapeutic oxygen may be produced by an oxygen concentrator using any suitable process such as Pressure Swing Adsorption (PSA). While devices which run on this principle are relatively inexpensive to manufacture and may reliably produce 87+% purified oxygen, they tend to be power inefficient. For example, conventional devices, such as the RESPIRONICS™ EVERFLO™ device, produces 5 LPM of 87+% Oxygen and consumes 300 watts (W) of power yielding 60 W/Liter (L) of purified oxygen. Portable oxygen concentrators tend to be more complex and expensive devices, but the refinement provides improved efficiency. For example, the conventional RESPIRONICS™ SIMPLYGO MINI™ device produces 1 LPM of 87+% Oxygen and consumes 40 W of power yielding 40 W/Liter of purified oxygen.

Considering that as general rule of thumb, the compressor needs to supply 15 L of air for every 1 L of oxygen produced and that the highest power consuming component in conventional oxygen concentrators is its compressor; it is desirable to minimize oxygen use so that the smallest possible compressor may be used.

Since conventional NOVDs utilize purified oxygen as the only gas source to drive air entrainment through the cannula and only 3/5 of the inhale period is therapeutic oxygen per the ISO standard, then assuming there are no other efficiency losses (such as oxygen left in the bronchioles, bronchial tubes, trachea, oral cavity, nasal cavity, or oxygen delivered past the inhale period), then 2/5 of all oxygen used by conventional NOVDs is wasted when the NOVD delivery setting covers the entire inspiratory period. As appreciated, the NOVD delivery setting may be shorter or longer than the entire inspiratory period. Translated to usage power, this equates to 16 W and 24 W, for the SIMPLYGO MINI™ and the EVERFLO™, respectively, of wasted power per liter of oxygen produced when the NOVD delivery setting covers the entire inspiratory period. Similarly, this translates to 6 LPM of wasted compressor air flow per liter of oxygen and thus the compressor must be larger, heavier and consume greater amounts of power to overcome this limitation. Regardless, this waste of resources results in units that are unnecessarily heavy and bulky with poor battery duration. This may unnecessarily limit mobility of those who depend upon these units for support, many of which are handicapped and/or disabled.

Limitations of Fixed Pulse Width

Conventional NOVDs may maintain a constant pulse width at each setting regardless of the breath rate of the patient, environmental conditions, sensor feedback, etc. This leads to poor therapeutic effect of the NIV or discomfort to the patient regardless of setting (e.g., Setting 1 through Setting 5, as may be typical).

For example, at Setting 1, an NOVD may have a pulse width of 430 ms. If the patient is breathing at 10 BrPM, then the inspiratory period (e.g., inhalation period) is 2000 ms per the ISO standard. This means that the positive pressure and NIV therapy would stop approximately one fifth of the way through the inhale, yielding incomplete lung fill and reduced therapy. Similarly, if the patient is breathing at 40 BrPM, then the inspiratory time is 500 ms per the ISO standard. This means that the positive pressure and NIV therapy would stop approximately prior to the completed inhale, yielding incomplete lung fill and reduced therapy. At Setting 1, the NOVD is producing partial therapy across breath rates of 10-40 BrPM.

At Setting 5, the NOVD may have a pulse width of 750 ms. If the patient is breathing at 10 BrPM, then the inspiratory period is 2000 ms per the ISO standard. This means that the positive pressure and NIV therapy would stop approximately one third of the way through the inhale, yielding incomplete lung fill and reduced therapy. Conversely, if the patient is breathing at 40 BrPM, then the inspiratory time is 500 ms per the ISO standard. This means that the positive pressure and NIV therapy would continue into the exhale and thus require the patient to exhale against the positive pressure besides the obvious waste of therapeutic oxygen delivered during the exhale. At Setting 5, the NOVD may only be producing ideal therapy at 27 BrPM. Below 27 BrPM, the NOVD may only be providing partial therapy and above 27 BrPM, the NOVD may be applying positive pressure against the exhale. This may lead to poor use of oxygen and patient discomfort as ideal ventilation therapy is not applied.

As a first example, US patent application n° 2009/183737 discloses a specified concentration of oxygen in a medical gas delivered to a patient by a gas delivery system which is achieved through the use of pulse width modulation control of the flows of one or more component medical gases. The flow of component medical gases can be controlled to improve the resolution and concentration range of medical gas delivered at low flow rates. A first flow of a first medical gas is provided and a second flow of a second medical gas is introduced to achieve a desired average medical gas concentration. When the second flow of the second medical gas is introduced, the first flow of the first medical gas is reduced to maintain a desired total medical gas flow rate.

As a second example, European patent application n° 2068992 describes modes, methods, systems and devices for providing assisted ventilation to a patient, including wearable ventilation systems with integral gas supplies, special gas supply features, ventilation catheters and access devices, and breath sensing techniques.

While oxygen gas is one type of medical gas that is delivered for a user, other medical gases such as medical air, nitrous oxide, etc., may also be delivered in respiratory applications yet the delivery systems suffer from at least some of the same problems discussed regarding oxygen delivery and therefore also would benefit from improvements in prior delivery systems.

Accordingly, embodiments of the present system overcome these and other disadvantages of conventional medical gas delivery including oxygen therapy devices such as conventional NOVDs.

## SUMMARY OF THE PRESENT SYSTEM:

The system(s), device(s), method(s), arrangements(s), interface(s), computer program(s), processes, etc., (hereinafter each of which will be referred to as system, unless the context indicates otherwise), described herein address problems in prior art systems. Embodiments of the present system may provide a system and method for a ventilation system that overcomes problems existing in prior systems. The invention is defined by the appended claims.

In accordance with embodiments of the present system, there is disclosed a ventilation device for providing a gas mixture for a user, the ventilation device including a compressor configured to output compressed air; an gas source configured to output medical gas; a gas source control valve configured to receive the compressed air and the medical gas and selectively output a gas flow including at least one of the medical gas and the compressed air; a patient interface configured to deliver the output gas flow of the gas source control valve; and a controller configured to determine an inspiratory period of the user, and control the gas source control valve to deliver substantially only the medical gas to the patient interface during a first portion of the inspiratory period and deliver substantially only the compressed air to the patient interface during a second portion of the inspiratory period. The medical gas source may include an oxygen concentrator which processes the compressed air to form oxygen gas as the medical gas. The ventilation device may include a proportional delivery valve configured to throttle the output gas flow of the gas source control valve that is delivered by the patient interface.

The controller may be configured to determine a switching time (Ts) in accordance with the determined inspiratory period of the user. The switch of the output gasses may occur at the switching time (Ts). When the medical gas is oxygen gas, the flow rate of the oxygen gas may be substantially equal to the flow rate of the compressed air. The flow rate of the oxygen gas and the compressed air may be controlled by a proportional delivery valve.

In addition, there is disclosed a ventilation device for providing a gas mixture for a user, the ventilation device including a compressor configured to output compressed air; a gas source control valve configured to receive the compressed air and medical gas and selectively output a gas flow including at least one of the medical gas and the compressed air; a conduit fluidly coupled to the gas source control valve for delivering the output gas flow to a user; and a controller configured to determine an inspiratory period of the user, and control the gas source control valve to deliver a first pulse of a first output gas flow and thereafter switch to deliver a second pulse of a second output gas flow during the inspiratory period. The first pulse may include oxygen gas as the medical gas and the second pulse may include the compressed air. The gas source control valve may have a first input coupled to the compressor and the device may include an oxygen concentrator coupled between the compressor and a second input of the gas source control valve. The oxygen concentrator may be configured to process the compressed air from the compressor to form and deliver the oxygen gas to the second input of the gas source control valve. The controller may be configured to determine a switching time (Ts) in accordance with the determined inspiratory period of the user. The controller may be configured to control the gas source control valve to terminate the first pulse and begin the second pulse at the determined switching time (Ts).

The controller may be configured to control the gas source control valve to deliver to the conduit a third pulse of the compressed air during an expiratory period that follows the first and second pulses. The controller may be configured to control the delivery of the third pulse at a lower flow rate than the first and second pulses. The flow rate of the first pulse may be substantially equal to the flow rate of the second pulse. The first and second pulse flows may be only output during the inspiration period and may be different in duration from each other.

Further, there is disclosed a ventilation device for providing a gas mixture for a user, the ventilation device including a compressor configured to output compressed air; an oxygen concentrator configured to process the compressed air to form and output oxygen gas; a gas source control valve configured to receive the compressed air and the oxygen gas and selectively deliver a first gas pulse comprising oxygen gas and a second gas pulse comprising compressed air; a patient interface configured to receive the first and second gas pulses delivered by the gas source control valve, and to deliver the first and second gas pulses; and a controller configured to determine a switching time (Ts) that occurs during an inspiratory period of a user, and control the gas source control valve to terminate the delivery of the first pulse and begin the delivery of the second pulse at the switching time (Ts). The controller may be configured to terminate the second pulse prior to the start of an expiratory period of the user.

The controller may be configured to control the gas source control valve to deliver to the patient interface a third gas pulse of the compressed air during an expiratory period that follows the first and second gas pulses.

The controller may be configured to control the delivery of the third pulse at a lower flow rate than the first and second pulses. The controller may be configured to determine the inspiratory period of the user. The controller may be configured to control the flow rate of the first gas pulse to be substantially equal to the flow rate of the second gas pulse. The patient interface may include an air entrainment interface.

## BRIEF DESCRIPTION OF THE DRAWINGS:

It should be expressly understood that the drawings are included for illustrative purposes and do not represent the scope of the present system. It is to be understood that the figures may not be drawn to scale. Further, the relation between objects in a figure may not be to scale and may in fact have a reverse relationship as to size. The figures are intended to bring understanding and clarity to the structure of each object shown, and thus, some features may be exaggerated in order to illustrate a specific feature of a structure. In the accompanying drawings, like reference numbers in different drawings may designate identical or similar elements, portions of similar elements and/or elements with similar functionality. The present system is explained in further detail, and by way of example, with reference to the accompanying drawings which show features of various exemplary embodiments that may be combinable and/or severable wherein:

FIG. 1 shows a block diagram of a portion of an oxygen concentrator (OC) system in accordance with embodiments of the present system;
FIG. 2 shows a block diagram of a portion of a system in accordance with embodiments of the present system;
FIG. 3 shows a block diagram of a portion of a system in accordance with embodiments of the present system;
FIG. 4 shows a block diagram of a portion of a system in accordance with embodiments of the present system;
FIG. 5 shows a block diagram of a portion of a system in accordance with embodiments of the present system;
FIG. 6 shows a block diagram of a portion of a system in accordance with embodiments of the present system;
FIG. 7 shows a block diagram of a portion of a system in accordance with embodiments of the present system;
FIG. 8 shows a block diagram of a portion of a system in accordance with embodiments of the present system;
FIG. 9 shows a block diagram of a portion of a system in accordance with embodiments of the present system;
FIG. 10 is a graph illustrating a comparison of a calculated ideal standard oxygen pulse from a portable oxygen concentrator at a setting intended to deliver 50 mL of oxygen vs. a split pulse delivery of the same amount of oxygen gas with a subsequent compressed air pulse and in accordance with embodiments of the present system;
FIG. 11 is a graph illustrating a standard blended gas delivery approach to deliver 250 ml of blended air and $O_2$ at 250 mL of blended air and $O_2$ at approximately 34 to 36% oxygen in accordance with embodiments of the present system; and
FIG. 12 is a graph illustrating a bi-level therapy mode in accordance with embodiments of the present system.

## DETAILED DESCRIPTION OF THE PRESENT SYSTEM:

The following are descriptions of illustrative embodiments that when taken in conjunction with the following drawings will demonstrate the above noted features and advantages, as well as further ones. In the following description, for purposes of explanation rather than limitation, illustrative details are set forth such as architecture, interfaces, techniques, element attributes, etc. However, it will be apparent to those of ordinary skill in the art that other embodiments that depart from these details would still be understood to be within the scope of the appended claims. Moreover, for the purpose of clarity, detailed descriptions of well-known devices, circuits, tools, techniques, and methods are omitted so as not to obscure the description of the present system.
The term and/or and formatives thereof should be understood to mean that only one or more of the recited elements may need to be suitably present (e.g., only one recited element is present, two of the recited elements may be present, etc., up to all of the recited elements may be present) in a system in accordance with the claims recitation and in accordance with one or more embodiments of the present system. In the context of the present embodiments, the terms "about", substantially and "approximately" denote an interval of accuracy that a person skilled in the art will understand to still ensure the technical effect of the feature in question which in some cases may also denote "within engineering tolerances". The term may indicate a deviation from the indicated numerical value of $\pm 20\%$, $\pm 15\%$, $\pm 10\%$, $\pm 5\%$, $\pm 1\%$ $\pm 0.5\%$ or $\pm 0.1\%$.
As used herein, the term patient may be interchangeably used with the term user.
As used herein, the term "air" is intended to denote a mix of gases, such as may be available as ambient air (e.g., atmospheric air which is a mix of typically 78% nitrogen and 21% oxygen with an extra 1% made up of a combination of carbon, helium, methane, argon and hydrogen), though the delivery system described may provide the air under pressure above ambient pressure.
As used herein, the term "medical gas" is intended to denote a medical gas that is therapeutically administered to a patient. While for purposes of simplifying the present discussion, oxygen gas is illustratively discussed, it is understood that in the examples, other medical gases such as medical air, nitrous oxide, etc., may be substituted in many instances for the oxygen gas without requiring further explanation or discussion. Accordingly, unless specified otherwise, the discussions that follow should be understood to include oxygen gas and/or these other medical gases.

Embodiments of the present system provide a ventilation system, such as an oxygen concentrator (OC) system (e.g., hereinafter system), which includes a gas flow path from a compressed air line to a delivery line with at least one valve for controlling switching between, or blending of, air (e.g., compressed air) and a medical gas, such as oxygen gas, in the delivery line, and/or for delivering either or both of the oxygen gas and compressed air to a patient via a patient interface, such as an air entraining cannula as a patient interface. In accordance with embodiments of the present system, another valve may be provided to deliver a gas to the patient, which gas may include a desired mix of gasses such as oxygen and/or air.

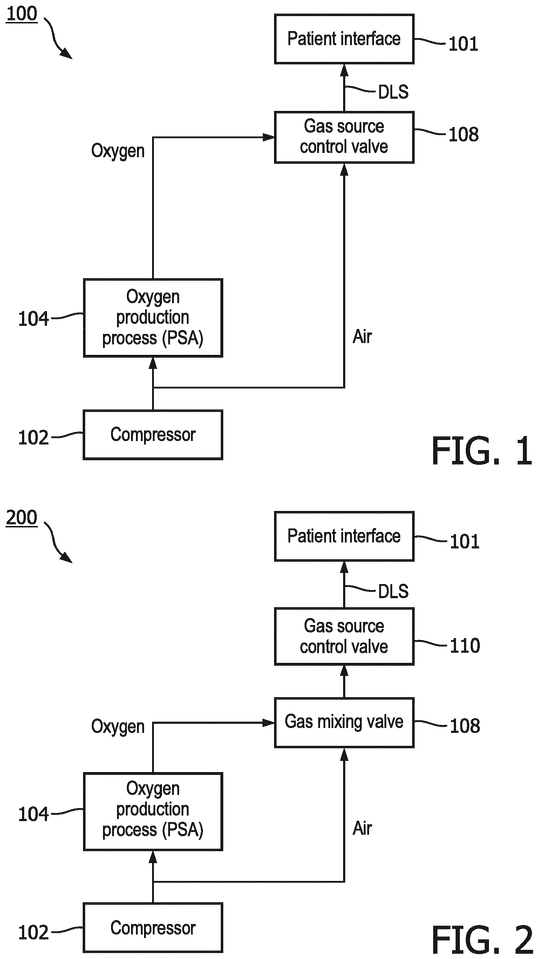

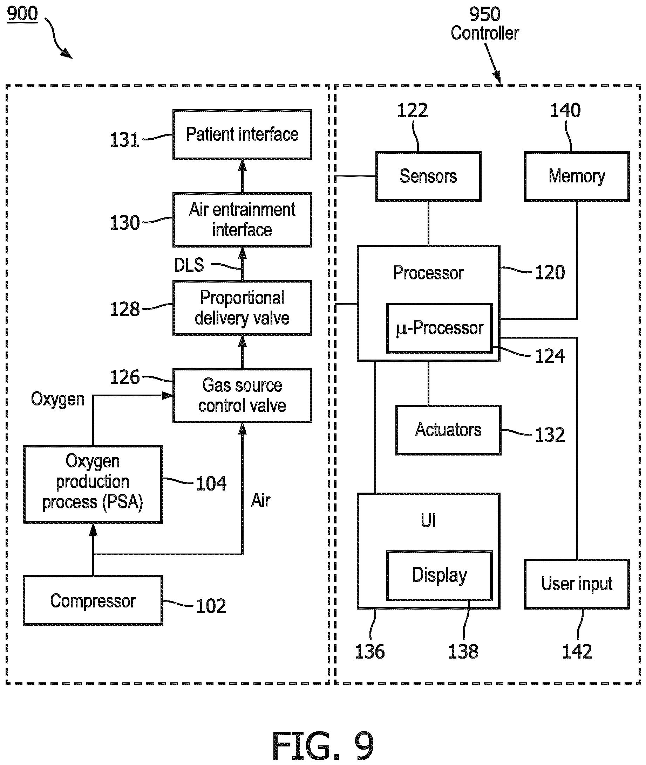

Several embodiments of oxygen concentrator systems will now be described with reference to Figures 1 through 9 with an illustrative detailed operational flow for each described below. With regard to the embodiments shown in Figures 1 through 8, it should be understood that these embodiments may include a controller or portions thereof as will be discussed in further detail below with reference to Figure 9.

FIG. 1 shows a block diagram of a portion of an oxygen concentrator (OC) system 100 (hereinafter system 100) in accordance with embodiments of the present system. Generally, the system 100 may include one or more of a compressor 102, an oxygen concentrator which may employ any suitable oxygen providing/production process(es) 104, illustratively shown as pressure swing adsorption (PSA) 104, a gas source control valve (GSCV) 108, and a patient interface 101.

The compressor 102 may be coupled (e.g., flow coupled) to the PSA 104 and the GSCV 108. The GSCV 108 may be flow coupled to a patient interface (PI) 101 via a distribution line set (DLS), such as a cannula. As utilized herein, the term patient interface (PI) should be understood to include a coupling on a therapeutic device that enables a connection to a further device that delivers gases to a patient. For example, a patient interface may include a coupling for a canula, air entrainment device, etc. In accordance with embodiments of the present system, the patient interface may include the delivery system (e.g., canula, air entrainment device, etc.).

The PI 101 may receive a gas mixture generated by the system to the patient via a cannula such as an air entrainment cannula. The gas mixture may include either or both of air (e.g., compressed air) and oxygen gas (e.g., concentrated oxygen gas) as may be determined by the system.

Sensors may sense system and/or biometric parameters (e.g., pulse rate, respiratory rate (e.g., in breaths per minute (BrPM)), etc. of a user), generate corresponding sensor information (e.g., gas pressure, airway pressure, temperature, gas analysis, BrPM, etc.), and provide this information to the controller for further processing. The controller in response, may control the system based on the sensor information to produce desired gas flow including desired gas pressure, airway pressure, gas composition (air and/or oxygen gas) and/or other desired gas flow characteristics. Further, the controller may operate selected valves, compressors, etc., to attain the desired gas flow characteristics. Additional valves of the system 100 may include actuators and/or may be coupled to actuators which may operate under the control of the controller to open and/or close or otherwise switch the corresponding valve(s) to attain the desired gas flow, pressure, etc., in accordance with embodiments of the present system. Valves of the system may be normally open (NO) or normally closed (NC) and the controller may control the valves accordingly as desired.

The compressor 102 may be operative under the control of the controller and may compress an ambient gas such as air and provide this compressed gas to both the PSA 104 and one or more valves of the system such as the GSCV 108.

The PSA 104 may employ any suitable process or method to generate concentrated oxygen from ambient air, such as the PSA process, and may be powered by any suitable power source or sources such as mains, capacitive, and/or battery sources. It is envisioned that the PSA may produce and output highly oxygen-enriched gas which may comprise approximately 87% to 96% oxygen ($O_2$) gas (hereinafter oxygen gas) which may be considered a highly concentrated oxygen gas. In operation, the PSA 104 may receive the compressed air from the compressor 102 and may process it to generate oxygen gas and may supply the concentrated oxygen to the GSCV 108. As readily appreciated, in accordance with any of the embodiments disclosed herein, oxygen may be provided by any other suitable source. Accordingly, while the term oxygen or oxygen gas is generally utilized herein, it is appreciated that any source of oxygen (e.g., 84% to 96% $O_2$ within gas stream) may be suitably utilized, such as concentrated oxygen or oxygen cannisters.

The GSCV 108 may then control switching between, and/or blending (e.g., mixing) of, the received oxygen gas and the received compressed air and thereafter output either or both of these gases alone or as a mix as an output gas to the PI 101 via the DLS for use by the patient. Thus, the GSCV 108 may be operative under the control of the controller of the system such as a processor (hereinafter both of which may be referred to as a processor for the sake of clarity) and may selectively control the flow, as well as mixture of, the compressed air or oxygen gas to the patient. In some embodiments, the processor may control the GSCV 108 based on gas pressure, airway pressure, and/or flow rates as may be desired.

While the PI 101 may be simply a coupling, the PI 101 may include a cannula (e.g., a patient cannula), such as an air entrainment cannula, which receives a gas flow via the (DLS), entrains ambient air, and feeds a mix of the received gas along with the entrained air to the patient. Thus, the air entrainment interface may use the gas from the DLS to entrain ambient air (e.g., room air) and may provide both of these gasses to the patient.

FIG. 2 shows a block diagram of a portion of a system 200 in accordance with embodiments of the present system. The system 200 may be similar to the system 100 and may include one or more of: the compressor 102, the PSA 104, the GSCV 108, a delivery valve 110 (e.g., gas source control valve) and the PI 101 and may operate similar to the system 100. The delivery valve 110 may be situated between and may be coupled to the GSCV 108 and the PI 101 (via the DLS) and may provide additional control of gas flow to the PI 101 via the DLS.

During operation, the GSCV 108 may control switching between, or blending of, compressed air and oxygen gas received from the compressor 102 and the PSA 104, respectively, to provide either of compressed air or oxygen gas, or produce a mixed gas which may be output to the delivery valve 110. The delivery valve 110 may, under the control of the processor, control the distribution of flow of this received gas to the PI 101 via the DLS for use by the patient.

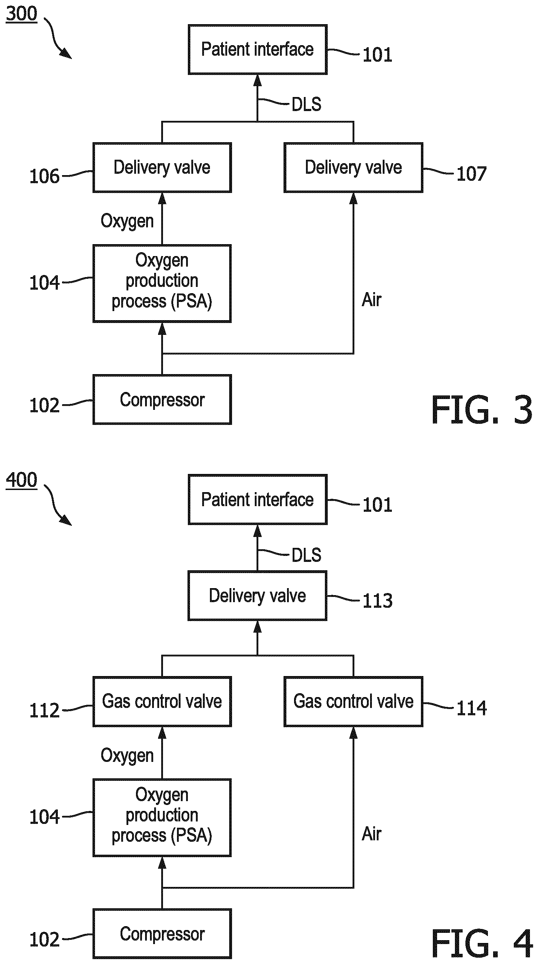

FIG. 3 shows a block diagram of a portion of a system 300 in accordance with embodiments of the present system. The system 300 may be similar to the systems 100 and 200 and may include the compressor 102, the PSA 104, first and second delivery valves 106 and 107, respectively, and the PI 101. The first delivery valve 106 may be situated between, and coupled to, the PI 101 and the PSA 104 and may be operative to control a flow of oxygen gas to the PI 101 via the DLS. The second delivery valve 107 may be situated between, and coupled to, the PI 101 and the compressor 103 and may be operative to control a flow of compressed air received from the compressor 102 to the PI 101 via the DLS. The DLS may include a "Y" coupling which gasses received from the first and/or second delivery valves 106 and 107, respectively, may be mixed before delivery to the PI 101 and delivery to the patient. The first and second delivery valves 106 and 107, respectively, may operate similar to the GSCV 108 of FIG. 1.

During operation, the first delivery valve 106 may control delivery of oxygen to the PI 101 and the second delivery valve 107 may control delivery of the compressed air to the PI 101. Each of the first and second delivery valves 106 and 107 may be switched (e.g., individually or together) or otherwise configured to provide either or both oxygen gas, compressed air, or a mixture of both, to the PI 101.

Additionally, both of the first and second delivery valves 106 and 107 may be switched (e.g., off) to stop, or otherwise prevent, the flow of oxygen and compressed air, respectively, to the PI 101.

FIG. 4 shows a block diagram of a portion of a system 400 in accordance with embodiments of the present system. The system 400 may be similar to the systems 100, 200, and 300 and may include the compressor 102, the PSA 104, first and second gas control valves 112 and 114, respectively, a gas delivery valve 113, and the PI 101. The first gas control valve 112 may be situated between, and coupled to, the delivery valve 113 and the PSA 104 and may be operative to control a flow of oxygen gas to the delivery valve 113. The second gas control valve 114 may be situated between, and coupled to, the delivery valve 113 and the compressor 103 and may be operative to control a flow of compressed air received from the compressor 102 to the delivery valve 113. The gas delivery valve 113 may then be operative to control a flow of compressed air received from the second gas control valve 114 to the PI 101 via the DLS and may be operative to control the flow of oxygen gas received from the first gas control valve 112 to the PI 101 via the DLS.

During operation, each of the first and second gas control valves 112 and 114, respectively, may be controlled to selectively provide oxygen gas and compressed air, respectively, to the delivery valve 113. The delivery valve 113 may then control the delivery of the gasses that it receives from one or more of: the first and second gas control valves 112 and 114, respectively, and may be operative to provide the received gases to the PI 101 via the DLS as discussed above.

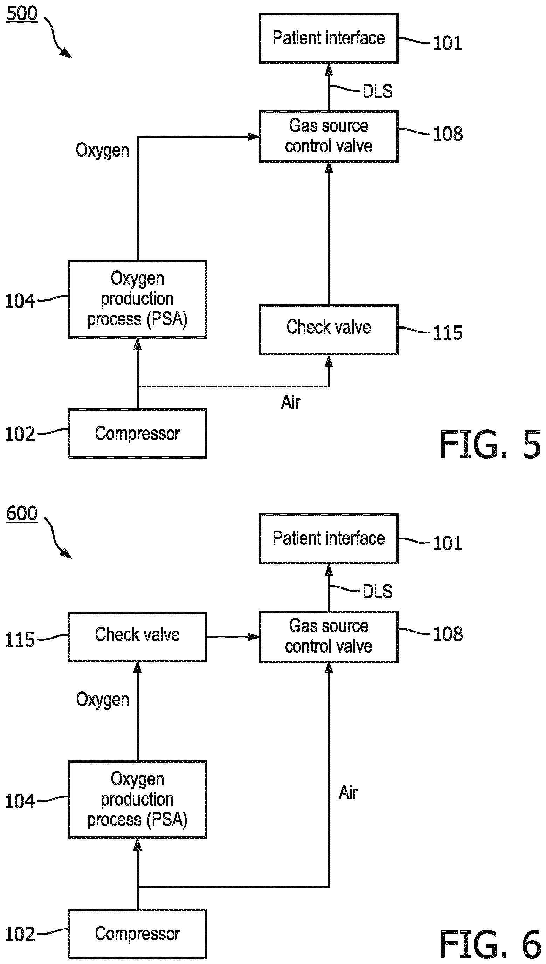

Illustrative embodiments in accordance with the present system with check valves will now be discussed with reference to FIGs. 5 and 6.

FIG. 5 shows a block diagram of a portion of a system 500 in accordance with embodiments of the present system. The system 500 may be similar to the systems 100 through 400 and may include one or more of: the compressor 102, the PSA 104, the GSCV 108, a check valve 115, and the PI 101 and may operate similar to systems 100 through 400 described above. The GSCV 108 may be coupled to, and may receive oxygen gas from, the PSA 104 and may receive compressed air from the compressor 102 via the check valve 115 which may be configured to prevent backflow of gasses from the GSCV 108 to the compressor 102 (which gas may, at times, include at least one of oxygen gas and compressed air). Accordingly, the GSCV 108 may be coupled to the compressor 102 via two parallel flow paths, one of which includes the PSA 104 and the other including the check valve 115. The GSCV 108 may then output one or more of: compressed air and oxygen gas to the PI 101 via the DLS. Although the check valve 115 is located in the compressed air flow path, it is appreciated that the oxygen gas flow path may also include a check valve such as the check valve 115. Such a configuration is shown and described with reference to FIG. 6 below.

FIG. 6 shows a block diagram of a portion of a system 600 in accordance with embodiments of the present system. The system 600 may be similar to the systems 100 and 500 and may include one or more of: the compressor 102, the PSA 104, the GSCV 108, the check valve 115, and the patient interface 101 and may operate similar to systems 100 through 500. The GSCV 108 may be coupled to, and receive oxygen from, the PSA 104 via the check valve 115 which may be configured to prevent backflow of gasses from the GSCV 108, such as oxygen gas and compressed air, and may receive oxygen from the PSA 104. The GSCV 108 may be coupled to the compressor 102 and may receive compressed air therefrom. The GSCV 108 may be coupled to the compressor 102 via two parallel flow paths one of which may be direct and the other of which may include the PSA 104 and the check valve 115 as may be desired. The GSCV 108 may output one or more of: compressed air, oxygen gas, and/or a combination of the two to the PI 101 via the DLS. As shown in the present embodiment, the check valve 115 may be in the oxygen gas flow path.

With reference to FIGs. 5 and 6, under normal operation of a pressure swing adsorption process of the PSA 104, the pressure output of the compressor 102 may cycle up and down and the check valves 115 in one or either gas flow path (e.g., oxygen or compressed air) may be operative to maintain the pressure of the corresponding gas flow path at peak compressor pressure even when the compressor outlet pressure drops during the PSA process. Maintaining a more consistent pressure on the delivery side allows for a more consistent delivery of gasses to the patient interface 101 and thus, the patient.

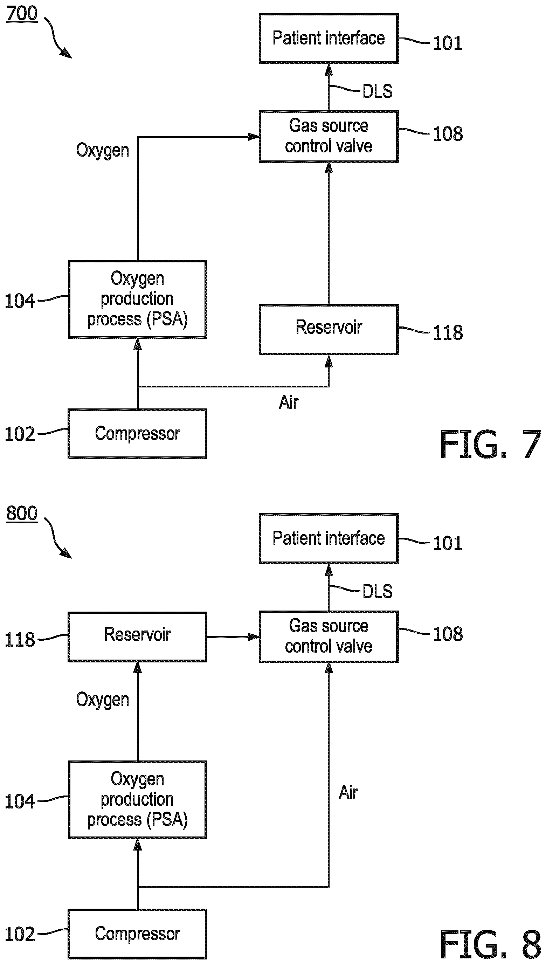

Embodiments with optional reservoirs will now be discussed with refence to FIGs. 7 and 8.

FIG. 7 shows a block diagram of a portion of a system 700 in accordance with embodiments of the present system. The system 700 may be similar to the system shown and described above such as the system 100 and may include one or more of: the compressor 102, the PSA 104, the GSCV 108, a reservoir 118, and the PI 101, and may operate similar to the system 100. The GSCV 108 may be coupled to, and may receive oxygen gas from, the PSA 104 and may receive compressed air from the compressor 102 via the reservoir 118. Accordingly, the GSCV 108 may be coupled to the compressor 102 via two parallel flow paths, one of which includes the PSA 104 and the other of which includes the reservoir 118. The GSCV 108 may then output one or more of: compressed air and oxygen gas to the PI 101 via the DLS. Thus, the reservoir 118 may be in the compressed air side gas flow path and may store compressed air for later use. The reservoir 118 may solve the problem of prior systems known in the art wherein gas delivered to a patient may vary in pressure, such as when the compressor cycles on and off. Further, in accordance with the present system, under normal operation of the PSA 104, the delivery pressure drops during delivery. A reservoir in one or either gas path, such as the reservoir 118, would serve to dampen the pressure drop during delivery. Maintaining a more consistent pressure on the delivery side allows for a more consistent delivery to the GSCV 108, and thereby to the patient interface 101. Further still, the reservoir 118 may act as a buffer which may enable the use of a smaller compressor 102 than may be necessary without it. However, as is appreciated, the reservoir 118 may also be in the oxygen gas flow path as will be described below with reference to FIG. 8.

FIG. 8 shows a block diagram of a portion of a system 800 in accordance with embodiments of the present system. The system 800 may be similar to the system 100 and 700 and may include one or more of: the compressor 102, the PSA 104, the GSCV 108, the reservoir 118, and the PI 101 and may operate similar to the systems 100 and 700. The GSCV 108 may be coupled to, and receive oxygen gas from, the PSA 104 via the reservoir 118 which may be configured to store oxygen gas from the PSA 104. The GSCV 108 may also be coupled to the compressor 102 and receive a compressed air supply therefrom. Accordingly, the GSCV 108 may be coupled to the compressor 102 via two parallel flow paths one of which may include the PSA 104 and the reservoir 118 and the other of which may be direct or may include another reservoir as may be desired. The GSCV 108 may output one or more of: compressed air and oxygen gas to the PI 101 via the DLS.

With reference to FIGs. 7 and 8, one or more optional reservoirs 118 may be situated in either or both of the oxygen and compressed air gas flow paths. During delivery of the corresponding gas (e.g., oxygen gas and/or compressed air), under normal operation of the compressor 102 and/or the PSA 104, delivery pressures output therefrom may vary over time for example, by dropping or swinging over time. The reservoirs may help to dampen this change during delivery of the corresponding gas or gasses. Maintaining a more consistent gas pressure and/or flow rates on the delivery side may provide for a more consistent delivery of a desired gas or gasses with more accurate and controlled results.

FIG. 9 shows a block diagram of a portion of a system 900 in accordance with embodiments of the present system. The system 900 may include one or more of: the compressor 102, the PSA 104, a GSCV 126, a proportional delivery valve 128 (hereinafter deliver valve which may be similar to the delivery valve 110 of FIG. 2), an air entrainment interface 130, and a patient interface (PI) 131 and may operate similar to the system 100 under of a controller 950. As may be readily appreciated, the air entrainment interface 130 is optional as the PI 131 may provide for suitable delivery of gase(s) to the patient. Further, the air entrainment interface 130 may be a portion of the PI 131 or may be simply coupled to the PI 131 when the system 900 is in use.

The controller 950 may control the overall operation of the system 900 and may include one or more of: a processor 120 which may include a microprocessor 124 and/or other hardware and/or software processing system(s), sensors 122, actuators 132, a user interface (UI) 136, a user input interface 142, and a memory 140. A more detailed illustrative description of the controller 950 is given below. It would be appreciated that in some embodiments the controller 950 may include digital and/or analog control circuitry (e.g., reservoirs, pressure-controlled valves, etc.) and may be suitably utilized or portions thereof, as a controller for other systems such as illustratively described regarding systems 100 through 800.

In accordance with the system 900, the compressor 102 may be flow coupled to, and provide compressed air to, both of the PSA 104 and the GSCV 126. The PSA 104 may receive the compressed air, form oxygen gas that is highly concentrated using any suitable process such as the PSA process and may then provide oxygen gas to the GSCV 126.

The GSCV 126 may receive the oxygen gas and the compressed air provided thereto, and, under control of the processor 120, selectively provide the received oxygen gas and/or compressed air to the proportional delivery valve 128.

The proportional delivery valve 128 may receive oxygen gas, compressed air, and/or a mixture of the two and may passively or actively (e.g., the latter of which may occur under the control of the processor 120) regulate pressure and/or flow of the received gas and output either or both (e.g., a mixture) of oxygen gas and compressed air. Thus, the GSCV 126 may selectively output one of: oxygen gas, compressed air, and a mixture of oxygen gas and compressed air under the control of the processor 120. The GSCV 126 may be switched during a BP so as to provide a first gas such as oxygen gas, a second gas such as compressed air, and/or a controlled mixture (passively or actively, such as under control of the processor 120) of these gasses during separate portions of a BP in accordance with a selected operating mode as will be discussed below.

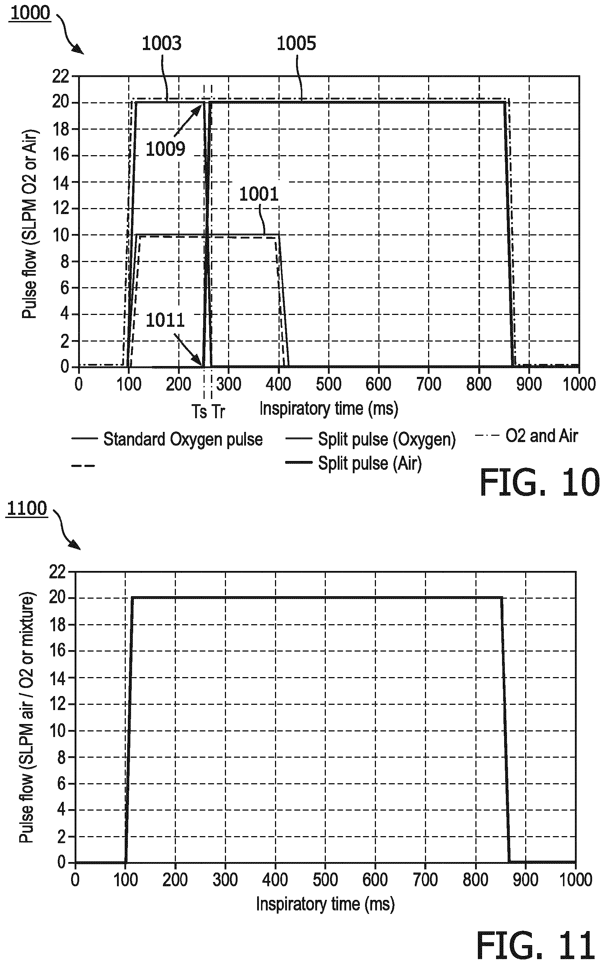

For example, during the split pulse delivery, the controller may control the GSCV 126 to switch (e.g., via control of one or more valves of the GSCV 126) to output oxygen gas (e.g., a first gas) during a first portion of an inspiratory period and, at a switchover time (Ts) (as will be discussed below with respect to FIG. 10), switch to output compressed air (e.g., a second gas different from the first gas) and terminate output of the oxygen gas (e.g., the first gas). The output gas flow of the GSCV may then be flow coupled to the air entrainment interface 130 via the proportional delivery valve 128 and the DLS creating a split pulse delivery in accordance with the present system as will be described below. It is envisioned that this switch may occur during an inspiratory period of a BP, for example determined by the system, as is illustratively described below with reference to FIG. 10.

The GSCV 126 may include one or more valves and/or mixers to selectively control the flow of one or more selected gasses such as compressed air (e.g., from the compressor), oxygen gas (e.g., from the PSA, etc.), and/or a combination of the two, to the proportional delivery valve 128 which may control flow and/or pressure of the gas flow output by the GSCV 126. In some embodiments, the GSCV 126 and the proportional delivery valve 128 may be integrated with each other.

During use, the processor 120 may obtain sensor information from, for example, a respiratory sensor (e.g., sensor 122) to determine a respiratory rate (e.g., BrPM), a start of an inspiratory cycle, duration of the inspiratory cycle, a start of an expiratory cycle, and/or a duration of the expiratory cycle of the patient. The processor 120 may then control the GSCV 126 to switch output gas flow type (as discussed above) between the two sources of gas (e.g., oxygen gas and compressed air) optionally in accordance with the sensor information (e.g., inspiratory period) and/or mode information (e.g., split pulse deliver mode selected), and may provide the output gas flow to the DLS via the optional proportional delivery valve 128. The air entrainment interface 130 may then entrain air using the received gas flow from the GSCV 126 and provide this combined flow (e.g., flow from the DLS and entrained air (e.g., room air) to the patient interface 131 for use by the patient.

In accordance with some embodiments, the GSCV 126 may be controlled (e.g., by the processor 120) to perform this switching based upon, for example, one or more of: predetermined volume (e.g., of one or more of the gasses), predetermined time, timing based on the user's determined BrPM, settings chosen for the proportional delivery valve 128, type or settings of the air entrainment interface 130, sensor information (e.g., feedback information, such as from an SPO2 sensor, etc.), inputs from the user (e.g., patient provides feedback as to how they are feeling during therapy), etc., as may be set by the system, therapist, patient, etc. Thus, the proportional delivery valve 128 may regulate (e.g., passively, actively, or semi-actively) a gas flow provided thereto and provide the regulated gas flow to the air entrainment interface 130.

The air entrainment interface 130 may be configured to direct the gas flow provided thereto to entrain ambient air using any suitable method or principle such as a venturi principle, etc., and may provide the entrained air flow (e.g., the gas flow from the DLS as well as the entrained air) to the patient via the PI 131. For example, assuming that current gas provided to the PI 131 is a pulse of oxygen (e.g., when the current gas flow is an oxygen gas flow), then the air entrainment interface 130 may utilize the venturi principle to entrain air into the pulse of oxygen provided thereto thus creating a larger flow and bolus which may then be delivered to the patient interface 101 which subsequently creates positive inspiratory pressure (IPAP) as the velocity of the gas slows upon entering the patient's airways.

The processor 120 may include one or more logic devices such as a microprocessor (e.g., μ-processor 124), a feedback circuit and/or the like and may receive sensor information from the one or more sensors 122.

The sensors 122 may include sensors such as flow sensors, pressure sensors, temperature sensors, altimeter, gas type/analysis sensors, bioinformatic sensors such as pulse oximetry, BrPM sensors, etc., and may sense parameters of the system coupled thereto (e.g., system 900 in the present illustrative example, though may be also be applicable to operation of the systems 100 through 800), may generate corresponding sensor information and provide the sensor information to the processor 120 for further analysis. The processor 120 may then control the system coupled thereto (e.g., system 900) in accordance with embodiments of the present system. For example, flow sensors may be utilized to determine flow into the patient interface 131 and pressure sensors may be utilized to ensure proper pressure is delivered to the patient to set/control system settings. In this way, flow and pressure sensors may be utilized to control the proportional delivery valve, reservoir(s), compressor and/or other valve(s)/systems sending flow into the patient interface, thereby ultimately creating patient airway pressure as desired. In this way, the present system is able to control the airway pressure to set levels, for example, based on a prescription.

A system bus may provide for power and signal flow within the system 900. Further, the system 900 may include a communication portion which may enable communication with other systems and/or portions thereof via one or more wired and/or wireless methods (e.g., Bluetooth™, WiFi™, 5G, 6G, etc.). The processor 120 may also communicate with one or more users via the user interface (UI) 136 which may include one or more of a display 138, such as a non-touch and/or a touch screen display, a speaker, an alarm, tactile feedback, lights, including lighting elements (e.g., colored indicator lights, etc.), etc. It is envisioned that the UI 136 may be locally or remotely located relative to the controller 950. The processor 120 may generate content and render this content of the UI 136. For example, the system may generate and render on the UI 136 mode selection items, pressure settings, flow settings, etc., for selection by a user. Then, the system may activate a corresponding mode, pressure, flow, etc., in response to determining that the user has made a selection (e.g., a selected a mode) using the UI 136. For example, a user may select from one or more modes described herein such as a pulse mode, etc., and the system upon detecting this selection, may apply the selected mode and operate in accordance with the selected mode. It is envisioned that operating instructions for the selected mode, operated on by the system 900, such as the controller 950, processor 120, etc., may be stored in a memory of the system such as the memory 140.

The processor 120 may further control the actuators 132 which may communicate with and/or control one or more valves of the system such as the proportional delivery valve 128, the GSCV 126, etc. The actuators 132 may, for example, adjust a setting of one or more flow and/or pressure regulators under the control of the processor 120. The processor 120 may further communicate with and/or control the compressor 102 and/or the PSA 104 in accordance with embodiments of the present system.

It should be appreciated that each of the valves described in these systems (e.g., systems 100 through 900) may function as binary (on/off) valves, or as valves which may control gas flow to a specific value (e.g., control gas flow rate), or as valves which may control flow to a specific pressure (e.g., control gas pressure), and/or a combination of any of these control methods. Accordingly, transducers and/or encoders (e.g., a rotary encoder) indicating status of a valve (e.g., on/off, setting, pressure setting, flow, temperature, gas type, saturation, etc.) may be provided to sense a status of a valve, for corresponding sensor information, and provide this sensor information to the processor 120 for further processing in accordance with embodiments of the present system.

It is envisioned that flow rates, pressures, and/or mixture in each gas flow path may be controlled passively with orifices, flow restriction elements, etc., or actively with, for example, valves, under the control of the processor 120. Accordingly, the valves and/or flow restrictors may be provided and may be controlled by the processor 120 in accordance with embodiments of the present system. The processor 120 may obtain sensor information, such as flow rate, flow pressure, flow temperature, as well as other parameters, such as patient airway pressure and pulse oximetry ($O_2$ delivered to the patient), etc., process this sensor information, and control one or more of the valves, regulators, pumps, and/or PSA in accordance with the sensor information and/or system settings such as the selected mode.

Gas flow, gas delivery volume, pressure, and/or gas delivery time in each gas flow path may be controlled through open-loop controls and methods, such as valve open times or set pressures, etc., or controlled through closed-loop feedback such as may be obtained from one or more sensors of the system such as: pressure sensors, flow sensors, pressure sensors, etc. However, it is also envisioned that analog control (pneumatic logic - fixed vessel sizes, etc.) methods may also be employed for open- or closed-loop control.

When operating in any continuous delivery mode, the device may deliver a pre-set gas flow rate, pressure, etc., or may vary the gas flow rate, pressure, etc., based on the patient's breathing pattern (e.g., BrPM, the start and/or length of the inspiratory cycle, the start and/or length of the expiratory cycle, etc.) and/or feedback from one or more sensors.

When operating in any pulsed delivery mode, the device may deliver to a pre-set pulse volume, pressure, etc., or may vary the pulse volume, pressure, etc., based on patient breathing patterns or feedback from sensors.

When operating in any blended gas delivery mode, the system may be controlled to deliver to a preset blend percentage, blend pressure, etc., or may vary the blend percentage, blend pressure, etc., based on patient breathing patterns (e.g., BrPM) or feedback from one or more sensors of the system such as a flow sensor.

The system may store information that it generates as well as operating instructions, user settings, system parameters, patient information, therapist settings, etc., in the memory 140 for later use under the control of the controller 950. It is further envisioned that authentication methods may be employed to authenticate a user for security purposes.

When operating in any separated gas delivery mode, the system may deliver either gas (e.g., compressed air and/or oxygen gas) to a pre-set volume, delivery time, pressure, and/or flow rate, or vary the volume, delivery time, pressure, and/or flow rate based on patient breathing patterns or feedback from one or more sensors of the system 900.

The device may mix delivery modes such as, but not limited to, continuous flow air (e.g., compressed air) with pulsed oxygen (e.g., oxygen gas), separated pulsed oxygen with pulsed blend (e.g., compressed air and oxygen gas), etc.

## MODES OF OPERATION

With further reference to FIGs. 1 through 9, the configuration of the systems 100 through 900 may each provide for operation in several possible modes such as a (a) Variable Pulse Width Mode; (b) Oxygen Therapy Mode; (c) NIV mode; (d) Oxygen Enriched NIV, Blended (OENIVB) Mode; and (e) Split Pulse Deliver Mode; each of which will be described in further detail below.

### Variable Pulse Width Mode

In this mode, the controller of the system may control the valves to deliver oxygen gas while the flow of compressed air is stopped and thereafter to deliver compressed air while the flow of oxygen gas is stopped since in accordance with the present system, one pulse of a gas may follow one pulse of another gas. In addition, the controller of the system may control the valves to stop the flow of oxygen gas and compressed air during a further interval, such as during all or a portion of an exhalation period. (E.g., see, 108, FIGs. 1 and 5-8; 108 and 110, FIG. 2; 106 and 107 FIG. 3; 101, 112 -114, FIG. 4; and 126 and 128, FIG. 9.) In this way, the present system has an ability to vary the pulse width of gas delivery (e.g., of oxygen gas and/or compressed air) based on one or more of device mode (e.g., split pulse delivery mode), device setting, patient breathing patterns, rate, and/or sensor feedback. Pulse width variability may provide for oxygen gas to be delivered in the most optimal portion of the inspiratory portion of a respiratory cycle (e.g.,

breathing period or breathing cycle) of the patient regardless of the respiratory rate (e.g., BrPM) of the patient. Likewise, variable pulse width allows for NIV to be more closely synced with the inspiratory period of the patient for improved comfort compared with conventional systems where positive pressure may stop before the inspiratory period has been completed, or positive pressure may continue past the inspiratory period and into the expiratory period. Accordingly, improved therapeutic benefits and patient comfort may be obtained.

### Oxygen Therapy Mode

In this mode, one or more valves (e.g., see, 108 FIGs. 1 and 5-8; 108 and 110, FIG. 2; 106 and 110 FIG. 3; 101, 112 and 114, FIG. 4; and 126 and 128, FIG. 9) are controlled such that flow of the compressed air is stopped and the flow of the oxygen gas (e.g., pressurized oxygen from the PSA 104) is provided continuously (e.g., during continuous flow) or selectively (e.g., during pulsed flow) to the DLS (e.g., via the delivery valve 130, FIG. 9 where applicable). In this mode, high-purity oxygen gas may be delivered to the patient via a cannula of the patient interface in continuous or pulsed flows under the control of the controller.

### NIV Mode

In this mode compressed air may be selectively provided to the DLS while oxygen gas is not. Accordingly, one or more valves controlling the flow of the compressed air to the DLS are either continuously opened (e.g., for continuous flow) or selectively opened (e.g., for pulsed flows) and one or more valves controlling the flow of the oxygen gas to the DLS are controlled to remain closed. Accordingly, the system may operate in the NIV mode and compressed air may be delivered to the patient via the patient interface with positive pressure via a patient interface, for example including an air entrainment cannula in continuous or pulsed flows.

### HFOT Mode

In this mode, one or more valves controlling the flow of the compressed air to the DLS are opened and one or more valves controlling the flow of the oxygen gas to the DLS are also opened so as to deliver high purity oxygen gas blended with compressed air to the patient via a cannula of the patient interface in continuous or pulsed flows at significantly higher flow rates than standard oxygen therapy may provide. Accordingly, HFOT may be delivered to the patient via an oxygen concentrator such as a PSA of the present system.

### OENIVB Mode

In this mode one or more valves which control the flow of the compressed air to the DLS are controllably opened and one or more valves controlling the flow of the oxygen gas to the DLS are also controllably opened such that a mix of compressed air and oxygen gas may be provided to the patient via the DLS and patient interface. The patient interface may utilize an air entrainment cannula and oxygen gas (e.g., of high purity) may be blended with compressed air and delivered to the patient at ventilation pressure in continuous or pulsed flows.

Split Pulse Delivery Mode

In this mode one or more valves controlling the flow of the compressed air (e.g., the GSCV 126, FIG. 9) are closed to stop, or otherwise prevent, the flow of compressed air to the DLS while one or more valves controlling the flow of the oxygen gas (e.g., from the GSCV 126, FIG. 9) to the DLS are open for a portion of the total delivery cycle, then these one or more valves toggle or switch such that the valve controlling the flow of the compressed air to the DLS is opened and the valve controlling the flow of the oxygen gas to the DLS is closed for the remainder of the total delivery cycle. The DLS may then deliver the gasses that it received to the patient interface via a patient interface such as an air-entrainment interface (e.g., an air entrainment cannula). In this mode the patient receives a more concentrated dose of oxygen at the initial portion of a breath NIV delivery where the Oxygen Capture Time is most relevant, then the remainder of the NIV delivery is made with compressed air only to reduce the burden on an oxygen supply providing the oxygen gas such as the PSA 104.

Thus, the system may deliver a pulse of gas to the patient that provides ventilatory pressure during the entire pulse, but where the pulse is split into an oxygen gas pulse during a first portion of a respiratory cycle and compressed air during the latter portion of the respiratory cycle. Accordingly, an oxygen concentrator such as the PSA may be employed as the gas source to supply oxygen gas as pulses to an air entrainment patient interface to deliver highly oxygen enriched gas (the output of the concentrator's PSA process is approximately 87% to 96% $O_2$) into the patient interface without the need for subsequent dilution with compressed gas as in the blended approach.

Thus, embodiments of the present system may employ a split pulse delivery mode in which the oxygen gas pulse is delivered first into the air entrainment interface followed by a compressed air pulse (or flow rate) to create the desired ventilatory IPAP but without intentional dilution of the oxygen. This may occur during an inspiratory period of a patient and prior to the expiratory period which follows, although may occur partially during the expiratory period, such as when the control is based on time (e.g., fixed time).

FIG. 10 is a graph 1000 illustrating a comparison of a calculated ideal standard oxygen pulse 1001 from a portable oxygen concentrator at a setting intended to deliver 50 mL of oxygen vs. a split pulse delivery of the same amount of oxygen gas with a subsequent compressed air pulse in accordance with embodiments of the present system. In this graph, a split pulse flow comprising first and second pulse flows 1003 and 1005, respectively, may be delivered during an inspiratory period in accordance with embodiments of the present system and may include first and second pulse flows of different gasses such as the first pulse flow 1003 of oxygen gas and the second pulse flow 1005 of compressed air. According during the inspiratory period, the system may provide a plurality of gas flows each of which may be different from, and substantially exclusive in time to, the other. For example, upon detecting the start of an inspiratory period (e.g., $T_0$= 100 ms in the present embodiments) the process may control one or more valves (e.g., 126 and 128 which may be controlled for each gas type) to flow oxygen gas (e.g., the first gas type) during a first portion of the inspiratory period (e.g., from $T_0$ until a switchover time Ts which occurs during the inspiratory period) and thereafter at Ts switch gas flow types to flow compressed air (e.g., the second gas type) during the second portion of the inspiratory period as illustrated by switchover points 1009 and 1011 at which points the valve(s) controlling the first pulse flow are shut to stop the first pulse flow 1003 of oxygen gas and the valve(s) controlling the second pulse flow are opened to flow compressed air. As appreciated, the start of the first pulse flows 1003 and the time Ts may be adjusted to account for oxygen and/or air that may be left in the system, such as in the cannula, at the beginning and/or end of the first and second pulse flows 1003 and 1005. A switchover period may be defined as a Tr-Ts, wherein Tr may denote a recover time at which the flow rate (e.g., in standard liters per minute (SLPM) of compressed air) may be substantially equal to the flow rate of the oxygen gas during the first pulse. This switchover period may be as small as possible. Further, during this switchover period, the flow of oxygen gas may decrease and the flow of compressed air may increase.

For example, the inspiratory period starts when the user begins to exert their respiratory muscles to draw air into the upper airways and ends when the user begins to exert their respiratory muscles to expel air from their airways. The period of the breath rate may be used to determine the expected inspiratory time, for example by assuming a typical I:E ratio such as 1:2, though other ratios may be suitably utilized or determined based on sensor data of a given user. In the illustrative example, a given breath rate of 20 BrPM would provide a breadth period of 3000 ms resulting in a breath once every 3 seconds. Assuming 1:2 for the I:E ratio, the user would have a 1000 ms inspiratory time and a 2000 ms expiratory time. Then using a reference such as 60% of the inspiratory time (referred to as oxygen capture time in the ISO standard) being viewed as therapeutic may provide an upper bound for TS. However, in accordance with the present system, there may be other tolerances and considerations, such as trigger delay (i.e., the time is takes from the onset of an inhalation to the time the onset of the inhalation can be detected, which may be on the order of 100 ms), that may lead you to set the TS shorter than the upper bound based on 60% of the inspiratory time. In accordance with embodiments of the present system, one could select from 33% to 50% of the inspiratory time to ensure delivering oxygen gas truly within the 60% timeframe, though other ranges may be envisioned. Further, the choice between something like 33% and 50% or some other number in that range may be decided based on peak flow rate (e.g., for patient comfort reasons you may not want the flow to exceed a certain level) or pressure requirements in your system (e.g., you may not have enough pressure to get the flow needed to achieve a very low delivery time %).

Although similar volumes of oxygen are provided, the split pulse concept of the present system contrasts with the calculated ideal standard oxygen pulse 1001 which only delivers a single gas which is oxygen gas. For example, the pulse flow 1001 delivers 50 mL of 87 to 96% oxygen. In contrast, in the split pulse flow, the pulse flow 1003 includes 50 mL of 87 to 96% oxygen gas delivered to the patient interface via the air entrainment interface, and the pulse flow 1005 delivers 200 mL of compressed air at approximately 21% oxygen (e.g., ambient air).

As seen in the graph 1000, each of the pulse flows 1003 and 1005, are performed at higher levels than the standard oxygen pulse 1001, and in doing such creates increased inspiratory positive airway pressure (IPAP) levels by the air entrainment interface. The IPAP delivered by the air entrainment interface may also be sustained until the end of the second pulse 1005, which is a compressed air pulse, and may be adjusted based on sensory information obtained by the system such as a breathing pattern of the patient and/or by predetermined settings of the system as may be factory set, set by a respiratory therapist, and/or by a patient as may be desired and/or authorized depending upon system settings. The higher levels of delivered gasses also assure that the pulse flows at switchover (e.g., after Ts and before Tr) do not fall below a threshold value such as 10 SLPM of oxygen gas or compressed air as shown. Further, each of the pulse flows may have substantially the same flow value. However, it is also envisioned that each of the pulse flows may have a different flow value from each other. Each of the pulses of the first and second pulses may have a different duration from each other. For example, from 0 to 30 SLPM oxygen gas and compressed air may be provided during each respective period and each of the gas flows may be the same or different from each other.

FIG. 11 which is a graph 1100 illustrating a standard blended gas delivery approach to deliver 250 ml of blended air and $O_2$ at 250 mL of blended air and $O_2$ at approximately 34 to 36% oxygen.

Comparing FIGs. 10 and 11, it is seen that embodiments of the present system may be more efficient than the standard blended approach as shown in graph 1100 wherein oxygen output by an oxygen concentrator is mixed and diluted by air from the PSA to create the total flow entering the air entrainment interface. If it is assumed that 50 mL of oxygen from the oxygen concentrator is blended with 200 mL of compressed air, a total pulse volume of 250 mL with a resulting oxygen purity of the gas entering the air entrainment interface is calculated at about 34 to 36%. Although this approach yields a similar total pulse volume as that obtained using the split pulse delivery of embodiments of the present system for example as shown in FIG. 10, the standard method does not provide for approximately about 87 to 96% oxygen purity into the air entrainment interface during the first portion of the patient's inspiration period which will allow for the majority of the delivered oxygen to enter the gas exchange region of their lungs. This process of the present system results in increased oxygen deliver to the patient resulting in enhanced comfort and mobility, all of which may be achieved while employing a smaller compressor and PSA and lower power usage than the conventional systems.

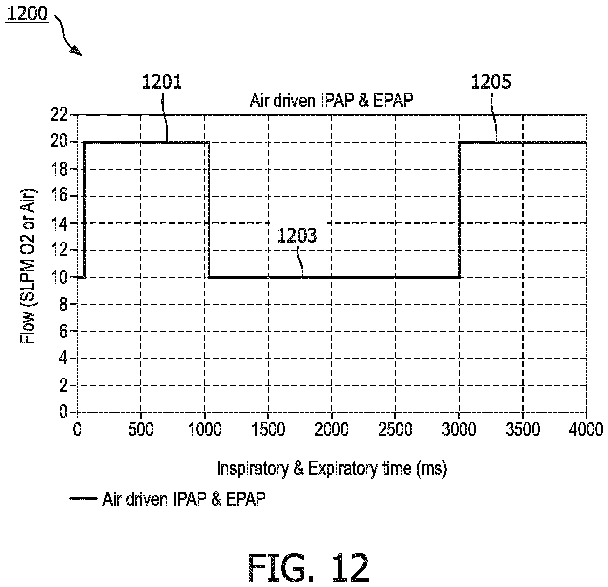

Embodiments of the present system may also be configured to only deliver compressed air to the air entrainment interface 130 for example by holding the GSCV 126 open to the compressor 102 and using the proportional delivery valve 128 to throttle a flow rate of the compressed air passing through to the air entrainment interface 130 to create bi-level (IPAP and expiratory positive airway pressure (EPAP) cycles) ventilation therapy for the patient via the patient interface 101 which may be in flow communication with the patient. As readily appreciated, other embodiments of the present system, such as shown in FIGs. 1-8, may also be suitably controlled to cycle between the IPAP and EPAP cycles. Illustrative timing and flow rates for compressed air only flows are illustrated with reference to FIG. 12 which is a graph 1200 illustrating timing and flow rates for only compressed air driven flows into the air entrainment interface to create IPAP and EPAP ventilation therapy cycles in accordance with embodiments of the present system. With reference to graph 1200, it includes first, second and third flows (e.g., cycles), 1201, 1203, and 1205, respectively, over a breathing cycle comprising inspiratory and expiratory periods as illustratively shown. With reference to the first flow 1201 and third flows 1205, these flows are performed at the beginning of an inspiration period (of two consecutive beathing cycles) and each delivers 20 SLPM to generate IPAP. With reference to the second flow 1203, this flow delivers 10 SLPM during an expiration period of the first breathing cycle. A pulse that is similar to the second flow 1203 may be repeated for the next expiration period which may follow pulse flow 1205. For each breathing cycle, this pattern may then be repeated to create 16 cm H2O IPAP and 6 cm H2O EPAP cycles, as an example, during this air driven NIV only mode. In accordance with embodiments of the present system, driving flows into the patient interface may illustratively be in the range of 0 to 30 SLPM to create the desired IPAP and EPAP levels in the range of 0 to 25 cm $H_2O$. It should be noted that while the ramp up/down pressure is illustratively shown in the figures as having a very steep increase/decrease (e.g., square wave shaped), in practice a ramp rate rise time and/or fall time may be less steep or may be another shape besides a ramp, such as an exponential rise time/fall time. In this way, the comfort of the patient may be improved when switching from one pressure to another and/or system capabilities may be accommodated.

Accordingly, embodiments of the present system may provide a portable oxygen concentrator system with a mode that may fully utilize the compressed air from a compressor to provide air driven IPAP and EPAP cycling using a lightweight and portable ventilation system. In this mode, depending upon settings, the system may be operative to temporarily suspend the PSA process (e.g., by turning the PSA 104 off) and use compressed air from the compressor in conjunction with the air entrainment interface to create both IPAP and EPAP for the patient. The PSA process may then be started again, for example, once there is a mode change which requires it.

In addition, while a bi-level therapy mode may utilize fully air driven ventilation (e.g., IPAP and EPAP cycles) as described above, however, a split pulse IPAP cycle driven first by an oxygen gas pulse followed by an air pulse may thereafter be followed by an air driven EPAP cycle during the expiratory period in accordance with embodiments of the present system. This split pulse IPAP cycle would allow for full ventilation therapy while also delivering oxygen during the most efficient and useful portion of the patient's inspiratory time.

## SYSTEM PORTABLITY AND POWER CONSUMPTION

Embodiments of the present system enhance system portability allowing users to experience greater mobility and comfort than conventional NOVDs. For example, by rebalancing gas flows between an oxygen delivery flow path and a compressed air flow path, a reduction in flow of the oxygen delivery flow path may be realized while enhancing flow of the compressed air flow path. This may reduce energy consumption and weight of the system. For example, by removing the burden of producing oxygen that is not therapeutically relevant, a significantly smaller compressor and/or smaller size sieve beds may be able to achieve the same total gas volume output compared to conventional NOVDs. By using a smaller compressor, cost, power, noise, and weight savings may be realized over prior systems. For example, to drive conventional NOVDs with up to SLPM of oxygen, a concentrator would need a compressor sized for 75 LPM

$$(5 \qquad 2 \cdot \frac{15\ LPM\ Air)}{1\ LPM\ O_2}]$$

In contrast, embodiments of the present system, when operating in modes which deliver oxygen substantially for the full clinically relevant portion of the inhale period, and thereafter switch to deliver only compressed air, would need a concentrator with a compressor sized for 47 LPM

$$(3 \qquad 2 \cdot \frac{15\ LPM\ Air}{1\ LPM\ O_2} + 2 \qquad ).$$

This is less than half the size of conventional compressors resulting in cost, weight, and energy savings over the prior systems.

## Oxygen Therapy & Efficiency Thereof

Embodiments of the present system allows for more efficient usage of delivered oxygen in NIV modes when compared to conventional NOVDs because only clinically relevant volumes of oxygen are being delivered to the patient during inspiration when oxygen transfer to the capillaries occurs. The on time, delivery volume, and flow rate of oxygen at the beginning of the delivery may be adjusted to provide the optimal therapeutic benefit while the remainder of the delivery may be driven by air via air entrainment to the patient interface.

## Variable Pulse Width

Embodiments of the present system provide improved therapeutic benefits and patient comfort over some prior systems, such as those that use preset timing, by allowing for the ability to vary the pulse width of delivery based on device mode, device setting, patient breathing patterns, respiratory rate, and/or other sensory feedback. Pulse width variability in accordance with the present systems allows the oxygen to be delivered in the most optimal portion of the inhale period regardless of the respiratory rate. Likewise, variable pulse width allows for NIV to be more closely synced with the patient's inhale period for improved comfort compared to NOVDs where positive pressure may stop before the inhale period is complete, or positive pressure may continue into the exhale period.

Further, various modes and configurations as described by embodiments of the present system may be applied to conventional oxygen concentrators resulting in non-invasive ventilation via the use of a patient interface that may include an air entrainment patient interface.

Referring back to the controller 950 of FIG. 9, this may represent a portion of a system for controlling a system (e.g., systems 100 through 900, etc.) or the like operating in accordance with embodiments of the present system. For example, a portion of the present system such as the controller 950 may include the processor 120 operationally coupled to the memory 140, the user interface (UI) 136 which may include a rendering device such as the display 138, the sensors 122, and the user input interface 142. The memory 140 may be any type of device for storing application data as well as other data related to the described operation. The application data and other data are received by the processor 120 for configuring (e.g., programming) the processor 120 to perform operation acts in accordance with the present system. The processor 120 so configured becomes a special purpose machine that is improved over prior systems (e.g., processors) and that is particularly suited for performing in accordance with embodiments of the present system.

The processor 120 may render the content such as still or video information on a UI of the system. This information may include information related to operating parameters, instructions, feedback, and/or other information related to an operation of the system or portions thereof. For example, the processor 120 may receive sensor information from one or more of: the sensors 122 and compare airflow information related to current airflow and/or pressure through one or more portions of the system (e.g., such as through the proportional delivery valve, the GSCV, etc.) with threshold airflow and/or pressure information and control the system accordingly. In this way, the processor 120 may determine whether the valves, pumps, PSAs, etc. is/are operating outside of operating parameters and may render results, such as on the UI 136 of this determination for the convenience of a user. The sensors 122 may include sensors of a system such as shown and/or portions thereof. Accordingly, the sensors 122 may sense related parameters, form sensor information, and provide this sensor information to the processor 120.

The user input interface 142 may include a keyboard, a mouse, a trackball, or other device, such as a touch-sensitive display, which may be stand alone or part of a system, such as part of a ventilator, an OC, an anesthesia device, a laptop, a personal digital assistant (PDA), a mobile phone (e.g., a smart phone), a smart watch, a smart phone, an e-reader, a monitor, a smart or dumb terminal or other device for communicating with the processor 120 via any operable link such as a wired and/or wireless communication link. The user input interface 142 may be operable for interacting with the processor 120 including enabling interaction within a UI 136 as described herein. Clearly the processor 120, the sensors 122, the memory 140, the UI 136, the display 138, and/or the user input device 142 may all or partly be a portion of a computer system or other device such as the OC, etc.

The methods of the present system are particularly suited to be carried out by a computer software program, such program containing modules corresponding to one or more of: the individual steps or acts described and/or envisioned by the present system. Such program may of course be embodied in a computer-readable medium, such as an integrated chip, a peripheral device or memory, such as the memory 140 or other memory coupled to the processor 120.

The program and/or program portions contained in the memory 140 may configure the processor 120 to implement the methods, operational acts, and functions disclosed herein. The memories may be distributed, for example between the clients and/or servers, or local, and the processor 120, where additional processors may be provided, may also be distributed or may be singular. The memories may be implemented as electrical, magnetic or optical memory, or any combination of these or other types of storage devices. Moreover, the term "memory" should be construed broadly enough to encompass any information able to be read from or written to an address in an addressable space accessible by the processor 120. With this definition, information accessible through a network is still within the memory, for instance, because the processor 120 may retrieve the information from the network for operation in accordance with the present system.

The processor 120 is operable for providing control signals and/or performing operations in response to input signals from the user input device 142 as well as in response to other devices of a network, such as the sensor(s) 122 and executing instructions stored in the memory 140. The processor 120 may include one or more of: a microprocessor, an application-specific and/or general-use integrated circuit(s), a logic device, etc. Further, the processor 120 may be a dedicated processor for performing in accordance with the present system and/or may be a general-purpose processor wherein only one of many functions operates for performing in accordance with the present system. The processor 120 may operate utilizing a program portion, multiple program segments, and/or may be a hardware device utilizing a dedicated or multipurpose integrated circuit.

The processor 120 may be operable to control one or more ventilation devices and/or other devices as described. Similarly, the processor 120 may be operable to control peripheral devices operating for example with the ventilator such as a humidifier and/or heater circuit operating in accordance with embodiments of the present system.

Accordingly, embodiments of the present system may provide a system to monitor the state of the ventilator and/or provide a user interface for the user to control settings and/or parameters of the ventilator using a local and/or remote communication. A wireless communication link such as a Bluetooth™ or Wi-Fi™ link between portions of the ventilator and the rendering device 138 and the system may enable rendering of system parameters on the UI 136 of the rendering device 138 which may also provide an entry area in which a user may change parameters such as ventilator settings, parameters, etc., of the system. Additionally, controller 950, or portions thereof, may be configured to link two or more ventilator systems using a two-way connection. With this connection, battery system parameters may be rendered and/or airflow rates, temperature, humidity, etc., may be displayed and/or adjusted by the user. Parameters such as temperature, pressures, flow, voltages, battery charge, specific oxygen ($Sp_{O2}$), carbon dioxide ($CO_2$), humidity, etc., may be determined and rendered on a UI of the system for the convenience of the user. Through the UI, the user may interact to select and/or change parameters in accordance with embodiments of the present system.

Embodiments of the present system may be configured to deliver High Flow Oxygen Therapy to a patient for example by using an oxygen concentrator with low power requirements to enhance mobility and ease of use. It is envisioned that embodiments of the present system may be operative with many existing oxygen products such as the Millennium M10™, EverFlo™, SimplyGo™, SimplyGo Mini™, and many other oxygen therapy devices as well as Home and Hospital Ventilation devices such as the V60™, Trilogy™, Trilogy Evo™, and E30™ devices. Embodiments of the present system may also be operative with continuous positive airway pressure (CPAP)/bilevel positive airway pressure (BiPAP) devices and other positive airway pressure devices.

Finally, the above discussion is intended to be merely illustrative of the present system and should not be construed as limiting the appended claims to any particular embodiment or group of embodiments. Thus, while the present system has been described with reference to exemplary embodiments, it should also be appreciated that numerous modifications and alternative embodiments may be devised by those having ordinary skill in the art, including using features that are described with regard to a given embodiment with other envisioned embodiments, without departing from the broader and intended scope of the present system as set forth in the claims that follow. For example, while the present system is illustratively described with reference to an oxygen concentrator system, such as a portable oxygen concentrator, as readily appreciated regarding the systems illustratively described and shown in the figures, the oxygen concentrator may readily be replaced with a different supply of medical gas (e.g., including medical air, oxygen, nitrous oxide, etc., including combinations thereof). For example, oxygen gas may be provided by oxygen cannisters, line oxygen, such as in a hospital or clinical setting, non-portable oxygen concentrator systems, etc., and still provide the benefits of the present system, for example, of reduced medical gas supply waste, reduced power waste, etc. Accordingly, each of the figures illustratively shown herein are considered to include or substitute such alternate medical gases and gas delivery systems as shown. For example, the compressor and oxygen production process illustratively shown in the figures may be replaced with an oxygen cannister and pressure regulator (or other medical gas/oxygen delivery system for that matter) without departing from the scope of the illustrative embodiments shown. Therefore, such alternatives should be considered within the scope of the present system. Further variations of the present system would readily occur to a person of ordinary skill in the art and are encompassed by the following claims.

In addition, any section headings included herein are intended to facilitate a review but are not intended to limit the scope of the present system. In addition, the specification and drawings are to be regarded in an illustrative manner and are not intended to limit the scope of the appended claims.

In interpreting the appended claims, it should be understood that:

a) the word "comprising" does not exclude the presence of other elements or acts than those listed in a given claim;
b) the word "a" or "an" preceding an element does not exclude the presence of a plurality of such elements;
c) any reference signs in the claims do not limit their scope;
d) several "means" may be represented by the same item or hardware or software implemented structure or function;
e) any of the disclosed elements may be comprised of hardware portions (e.g., including discrete and integrated electronic circuitry), software portions (e.g., computer programming), and any combination thereof;
f) hardware portions may be comprised of one or both of analog and digital portions;
g) any of the disclosed devices, features and/or portions thereof may be combined together or separated into further portions unless specifically stated otherwise;
h) no specific sequence of acts or steps is intended to be required unless specifically indicated; and
i) the term "plurality of" an element includes two or more of the claimed element, and does not imply any particular range of number of elements; that is, a plurality of elements may be as few as two elements, and may include an immeasurable number of elements.

## Claims

1.    A ventilation device (100) for providing a gas mixture for a user, the ventilation device comprising:

a compressor (102) configured to output compressed air;
an oxygen concentrator (104) configured to process the compressed air to form and output oxygen gas;
a gas source control valve (108) configured to receive the compressed air and the oxygen gas and selectively deliver a first gas pulse comprising oxygen gas and a second gas pulse comprising compressed air;

a patient interface (101) configured to receive the first and second gas pulses delivered by the gas source control valve, and to deliver the first and second gas pulses; and

a controller (950) configured to:

determine a switching time that occurs during an inspiratory period of a user, and control the gas source control valve to terminate the delivery of the first pulse and begin the delivery of the second pulse at the switching time, and

to terminate the second pulse prior to the start of an expiratory period of the user,

**characterized in that** the controller (950) is configured to control the gas source control valve to deliver to the patient interface a third gas pulse of the compressed air during an expiratory period that follows the first and second gas pulses, wherein the controller (950) is configured to control the delivery of the third pulse at a lower flow rate than the first and second pulses.

2. The ventilation device of claim 1, wherein the controller (950) is configured to determine the inspiratory period of the user.

3. The ventilation device of claim 1, wherein the controller (950) is configured to control the flow rate of the first gas pulse to be substantially equal to the flow rate of the second gas pulse.

4. The ventilator device of claim 1, wherein the patient interface includes an air entrainment interface (130).

**Patentansprüche**

1. Beatmungsvorrichtung (100) zum Bereitstellen eines Gasgemisches für einen Benutzer, wobei die Beatmungsvorrichtung umfasst:

einen Kompressor (102), der so konfiguriert ist, dass er Druckluft ausgibt;

einen Sauerstoffkonzentrator (104), der so konfiguriert ist, dass er die Druckluft verarbeitet, um Sauerstoffgas zu bilden und auszugeben;

ein Gasquellen-Steuerventil (108), das so konfiguriert ist, dass es die Druckluft und das Sauerstoffgas empfängt und selektiv einen ersten Gasstoß, der Sauerstoffgas umfasst, und einen zweiten Gasstoß, der Druckluft umfasst, abgibt;

eine Patientenschnittstelle (101), die so konfiguriert ist, dass sie den ersten und den zweiten Gasstoß, die vom Gasquellen-Steuerventil abgegeben werden, empfängt und den ersten und den zweiten Gasstoß abgibt; und eine Steuereinheit (950), die so konfiguriert ist, dass sie:

einen Umschaltzeitpunkt, der während einer Einatmungsperiode eines Benutzers auftritt, bestimmt und das Gasquellen-Steuerventil so steuert, dass es zum Umschaltzeitpunkt die Abgabe des ersten Stoßes beendet und die Abgabe des zweiten Stoßes beginnt, und

den zweiten Stoß vor dem Start einer Ausatmungsperiode des Benutzers beendet,

**dadurch gekennzeichnet, dass** die Steuereinheit (950) so konfiguriert ist, dass sie das Gasquellen-Steuerventil so steuert, dass es während einer Ausatmungsperiode, die auf den ersten und den zweiten Gasstoß folgt, einen dritten Gasstoß der Druckluft an die Patientenschnittstelle abgibt, wobei die Steuereinheit (950) so konfiguriert ist, dass sie die Abgabe des dritten Stoßes mit einer niedrigeren Strömungsrate als den ersten und den zweiten Stoß steuert.

2. Beatmungsvorrichtung nach Anspruch 1, wobei die Steuereinheit (950) so konfiguriert ist, dass sie die Einatmungsperiode des Benutzers bestimmt.

3. Beatmungsvorrichtung nach Anspruch 1, wobei die Steuereinheit (950) so konfiguriert ist, dass sie die Strömungsrate des ersten Gasstoßes so steuert, dass sie im Wesentlichen gleich der Strömungsrate des zweiten Gasstoßes ist.

4. Beatmungsvorrichtung nach Anspruch 1, wobei die Patientenschnittstelle eine Lufteintragsschnittstelle (130) beinhaltet.

**Revendications**

1. Dispositif de ventilation (100) pour fournir un mélange gazeux à un utilisateur, le dispositif de ventilation comprenant :

un compresseur (102) configuré pour délivrer en sortie de l'air comprimé ;

un concentrateur d'oxygène (104) configuré pour traiter l'air comprimé afin de former et de délivrer en sortie de l'oxygène gazeux ;

une vanne de commande de source de gaz (108) configurée pour recevoir l'air comprimé et l'oxygène gazeux et délivrer sélectivement une première impulsion de gaz comprenant de l'oxygène gazeux et une seconde impulsion de gaz comprenant de l'air comprimé ;
une interface de patient (101) configurée pour recevoir les première et deuxième impulsions de gaz délivrées par la vanne de commande de source de gaz, et pour délivrer les première et deuxième impulsions de gaz ; et un dispositif de commande (950) configuré pour :

déterminer un temps de commutation qui se produit pendant une période d'inspiration d'un utilisateur, et commander la vanne de commande de source de gaz pour terminer la délivrance de la première impulsion et commencer la délivrance de la deuxième impulsion au temps de commutation, et terminer la deuxième impulsion avant le début d'une période d'expiration de l'utilisateur,

**caractérisé en ce que** le dispositif de commande (950) est configuré pour commander la vanne de commande de source de gaz afin de délivrer à l'interface de patient une troisième impulsion de gaz de l'air comprimé pendant une période d'expiration qui suit les première et deuxième impulsions de gaz, dans lequel le dispositif de commande (950) est configuré pour commander la délivrance de la troisième impulsion à un débit inférieur aux première et deuxième impulsions.

2. Dispositif de ventilation selon la revendication 1, dans lequel le dispositif de commande (950) est configuré pour déterminer la période d'inspiration de l'utilisateur.

3. Dispositif de ventilation selon la revendication 1, dans lequel le dispositif de commande (950) est configuré pour commander le débit de la première impulsion de gaz pour qu'il soit sensiblement égal au débit de la deuxième impulsion de gaz.

4. Dispositif de ventilation selon la revendication 1, dans lequel l'interface de patient inclut une interface d'entraînement d'air (130).

100

FIG. 1

200

FIG. 2

300

Patient interface —101

DLS

106— Delivery valve          Delivery valve —107

Oxygen

104— Oxygen production process (PSA)

Air

102— Compressor

**FIG. 3**

400

Patient interface —101

DLS

Delivery valve —113

112— Gas control valve          Gas control valve —114

Oxygen

104— Oxygen production process (PSA)

Air

102— Compressor

**FIG. 4**

FIG. 5

FIG. 6

700

| | |
|---|---|
| | Patient interface — 101 |

↑ DLS

| | |
|---|---|
| Oxygen → | Gas source control valve — 108 |

104 — Oxygen production process (PSA)

Reservoir — 118

102 — Compressor

Air

# FIG. 7

800

| | |
|---|---|
| | Patient interface — 101 |

↑ DLS

118 — Reservoir → Gas source control valve — 108

↑ Oxygen

104 — Oxygen production process (PSA)

Air

102 — Compressor

# FIG. 8

900

950
Controller

131 — Patient interface

130 — Air entrainment interface

DLS

128 — Proportional delivery valve

126 — Gas source control valve

Oxygen

104 — Oxygen production process (PSA)

Air

102 — Compressor

122 — Sensors

140 — Memory

Processor — 120

μ-Processor — 124

Actuators — 132

UI

Display

136        138

User input

142

FIG. 9

FIG. 10

FIG. 11

FIG. 12

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63220595
- US 2009183737 A
- EP 2068992 A